# EUROPÄISCHE PATENTSCHRIFT

(11) **EP 3 612 620 B1**
(45) Veröffentlichungstag und Bekanntmachung des Hinweises auf die Patenterteilung: **03.03.2021**
(21) Anmeldenummer: 17719552.6
(22) Anmeldetag: 21.04.2017
(51) Int. Cl.: C11B 9/00, C11D 3/50, C07C 255/07

(54) **4-ETHYL-OCTEN-2/3-NITRIL ALS RIECHSTOFF**
4-ETHYL-OCTENE-2/3-NITRILE AS A FRAGRANCE
4-ÉTHYL-OCTÈNE-2/3-NITRILE S'UTILISANT COMME SUBSTANCE ODORANTE

(43) Veröffentlichungstag der Anmeldung: 26.02.2020
(73) Patentinhaber: Symrise AG, 37603 Holzminden (DE)
(72) Erfinder: HÖLSCHER, Bernd, 37620 Halle (DE); WAGNER, Tobias, 37627 Hellental (DE)
(74) Vertreter: Eisenführ Speiser
(86) Internationale Anmeldenummer: PCT/EP2017/059479
(87) Internationale Veröffentlichungsnummer: WO 2018/192665

(56) Entgegenhaltungen:
- EP-A1- 1 413 570
- EP-A2- 0 395 982
- WO-A1-2008/037105
- US-A- 2 352 515
- US-A- 4 156 690
- US-A1- 2010 279 917
- JAMES W. WILT ET AL: "Cyanomethylidenebis(triphenylphosphonium) Dibromide. Its Use in a Convenient Modification of the Wittig Reaction", JOURNAL OF ORGANIC CHEMISTRY, Bd. 36, Nr. 14, 1. Januar 1971 (1971-01-01), Seiten 2026-2027, XP055379320,
- YOSHIRO SATO AND YASUKO NIINOMI: "Selective Synthesis of (Z)-Alk-2-enenitriles from Aldehydes", JOURNAL OF THE CHEMICAL SOCIETY, CHEMICAL COMMUNICATIONS, Nr. 1, 1982, Seiten 56-57, XP002771374,

## Beschreibung

Die vorliegende Erfindung betrifft primär die Verwendung von 4-Ethyl-octen-2-nitril und/oder 4-Ethyl-octen-3-nitril, d.h. einer Verbindung der Formel (I) wie hierin beschrieben, als Riechstoff. Die Erfindung betrifft zudem neue Riechstoffkompositionen umfassend 4-Ethyl-octen-2/3-nitril, parfümierte Artikel umfassend 4-Ethyl-octen-2/3-nitril sowie diverse Verfahren zum Vermitteln, Modifizieren und/oder Verstärken bestimmter Geruchsnoten.

Weitere Aspekte und bevorzugte Ausgestaltungen der vorliegenden Erfindung ergeben sich aus den nachfolgenden Ausführungen, den beigefügten Beispielen und insbesondere den beigefügten Patentansprüchen.

EP 1 413 570 A1 offenbart die Verbindung 5,7,7-Trimethyloctannitril sowie bestimmte Verwendungen dieser Verbindung, insbesondere als (Iris-)Riechstoff.

US 2010/0279917 A1 offenbart Verbindungen der allgemeinen Formel 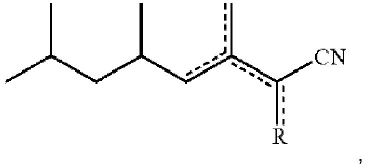 in der R ein Wasserstoffatom, eine Methylgruppe, eine CH₂OH-Gruppe, eine Estergruppe oder eine Methylengruppe darstellt, und in der nicht mehr als eine der vier gestrichelten Linien eine Kohlenstoff-Kohlenstoff-Doppelbindung darstellt, einen Prozess zu deren Herstellung und ihren Einsatz als Riechstoffe in Parfümzusammensetzungen.

US 4,156,690 A offenbart alpha, beta-Dialkyl konjugierte Nitrile der allgemeinen Formel 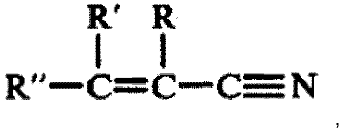 die interessante Parfümeigenschaften aufweisen. R stellt dabei ein aliphatisches Kohlenwasserstoffradikal mit 1 bis 15 Kohlenstoffatomen dar, das keine ungesättigte Verbindung in Konjugation mit dem Nitril oder der alpha, beta-olefinischen ungesättigten Verbindung aufweist. R' und R" sind gleich oder verschieden voneinander und entsprechen der Definition von R, oder R' und R", zusammen genommen, bilden ein cycloaliphatisches Radikal mit 6 bis 15 Kohlenstoffatomen.

WO 2008/037105 A1 offenbart die Verwendung kurzkettiger alpha, beta-ungesättigter Nitrile als Geschmacks- und Geruchsstoffe.

EP 0 395 982 A2 offenbart beta, gamma-ungesättigte Nitrile, deren Herstellung und Verwendung als Riechstoffe.

Trotz einer Vielzahl bereits vorhandener Riechstoffe besteht in der Parfümindustrie auch weiterhin ein genereller Bedarf an neuen Riechstoffen. So besteht beispielsweise ein Bedarf an Riechstoffen, die in der Lage sind (in Riechstoffkompositionen) neben einer primären Duftnote weitere interessante Noten zu erzeugen und mit ihren neuartigen bzw. originellen olfaktorischen Eigenschaften die Möglichkeiten des Parfümeurs zu erweitern. Insbesondere besteht ein Interesse an Riechstoffen mit Duftnoten, welche in der Lage sind, eine harmonische Kombination mit blumigen und/oder fruchtigen Riechstoffen einzugehen. Vorzugsweise sollte dabei eine Überlagerung der unterschiedlichen geruchlichen Aspekte und Noten erfolgen, um dadurch einen insgesamt komplexen Geruchseindruck zu erzeugen.

Für die Kreation neuartiger Kompositionen besteht ständiger Bedarf an Riechstoffen mit besonderen sensorischen Eigenschaften, die geeignet sind, als Grundlage für die Komposition von neuartigen Parfüms mit komplexem sensorischen Charakter zu dienen. Bevorzugte gesuchte Riechstoffe sollten neben einer bestimmten Note weitere Noten und Aspekte aufweisen, die ihnen Charakter und Komplexität verleihen.

Die Suche nach geeigneten Stoffen, die zur vorliegenden Erfindung führte, wurde durch folgende Sachverhalte erschwert:
- die Mechanismen der Geruchswahrnehmung sind nicht ausreichend bekannt;
- die Zusammenhänge zwischen der speziellen Geruchswahrnehmung einerseits und der chemischen Struktur des zugehörigen Riechstoffs andererseits sind nicht hinreichend erforscht;
- häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Die primäre Aufgabe bestand nun darin, Riechstoffe zu finden, die ein interessantes, vorzugsweise komplexes, und originelles sensorisches Profil aufweisen und sich als Riechstoffe für den Einsatz in z.B. der Parfümerie oder anderen Gebieten, ggf. auch in verzehrbaren Zubereitungen, eignen.

Es wurden im Rahmen der vorliegenden Erfindung insbesondere Stoffe gesucht, die eine, mehrere oder sämtliche der Noten grün, blumig und holzig aufweisen bzw. vermitteln, modifizieren und/oder verstärken können. Insbesondere sollten Stoffe gefunden werden, welche vorzugsweise zumindest eine der Noten grün, nitrilig, rosig nach Iris und Karotte aufweisen und/oder vermitteln können.

Die gesuchten Stoffe sollten die Herstellung von neuartigen Riechstoffkompositionen mit besonderen geruchlichen Noten und Aspekten ermöglichen. Von Vorteil wären dabei solche Stoffe, welche insbesondere zur Kombination mit weiteren Riechstoffen geeignet sind, die eine oder mehrere der Noten grün, nitrilig und/oder rosig nach Iris- und Karottennote aufweisen.

Daneben sollten die diese primäre Aufgabe erfüllenden Riechstoffe vorzugsweise über ihre primären, nämlich geruchlichen, Eigenschaften hinaus zusätzliche positive Sekundäreigenschaften besitzen, wie z.B. eine hohe Stabilität unter bestimmten Anwendungsbedingungen, eine hohe Ausgiebigkeit, ein gutes Haftungsvermögen, eine hohe Substantivität oder geruchsverstärkende Eigenschaften (sogenannte Booster- oder Enhancer-Wirkung), und/oder in Kombination mit anderen Riechstoffen deren Natürlichkeit, Frische, Fülle, (Strahl-)Kraft und/oder Ausstrahlung abrunden, so dass sensorisch bemerkenswerte Effekte erzielt werden können.

Erfindungsgemäß gelöst wird die primäre gestellte Aufgabe durch die Verwendung von 4-Ethyl-octen-3-nitril (4-Ethyl-oct-3-en-nitril; Formel (la); s. z.B. CAS-Nr. 29127-85-3) und/oder 4-Ethyl-octen-2-nitril (4-Ethyl-oct-2-en-nitril; Formel (Ib); s. z.B. CAS-Nr. 82125-04-0), d.h. einer oder mehrerer bzw. beider Verbindung(en) der Formel (I) (4-Ethyl-octen-2/3-nitril) 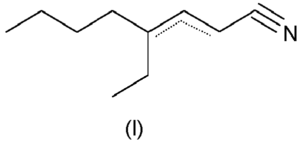 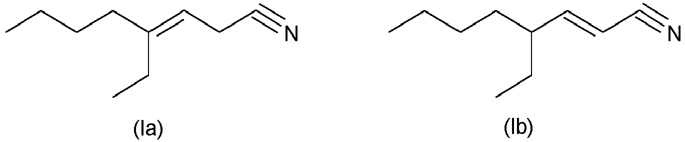 als Riechstoff (bzw. Riechstoffmischung). Im Rahmen der vorliegenden Erfindung kann eine Verbindung der Formel (la), eine Verbindung der Formel (Ib) oder - bevorzugt - eine Mischung einer Verbindung der Formel (la) und einer Verbindung der Formel (Ib) verwendet bzw. eingesetzt werden. Eine erfindungsgemäß einzusetzende Verbindung der Formel (I) bzw. (la) oder (Ib) kann in beliebiger stereoisomerer Form, d.h. Konfiguration (Enantiomere, cis/trans-Isomere) bzw. Konformation vorliegen bzw. kann als beliebige Mischung von Stereoisomeren vorliegen (z.B. E/Z-Gemisch).

Sofern in Rahmen dieses Textes versehentlich eine Unstimmigkeit zwischen dem chemischen Namen und den dargestellten Strukturformeln auftreten sollte, gelten die dargestellten Strukturformeln.

Das hierin für eine Verbindung der Formel (I) Gesagte, insbesondere die hierin beschrieben Vorteile, gelten besonders auch für die bevorzugt zu verwendende bzw. einzusetzende Mischung einer Verbindung der Formel (la) und einer Verbindung der Formel (Ib) (s. oben).

Eine Verbindung der Formel (I) verfügt über ganz eigenständige olfaktorische Eigenschaften, die sich deutlich von den bekannten Riechstoffen abheben und diese auch übertreffen. Die Eignung der Verbindungen der Formel (I) als Riechstoffe war bislang nicht bekannt. Es ist daher besonders überraschend, dass auf dem bereits gut untersuchten Gebiet ein Riechstoff mit wertvollen, interessanten und komplexen Geruchseigenschaften gefunden werden konnte.

Vorzugsweise betrifft die erfindungsgemäße Verwendung eine Verwendung zum Vermitteln, Modifizieren und/oder Verstärken einer oder mehrerer Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens einer der Geruchsnoten ausgewählt aus der Gruppe bestehend aus den Noten grün, nitrilig, rosig und Iris- und Karottennoten, bevorzugt rosig mit Iris- und Karottennoten.

Die Tatsache, dass eine erfindungsgemäß einzusetzende Verbindung der Formel (I) einen sehr komplexen und vielfältigen sensorischen Gesamteindruck zu vermitteln mag, der sonst meist nur von Mischungen mehrerer Komponenten (wie beispielsweise ätherischen Ölen oder Gewürzmischungen) erreicht werden kann, ist besonders überraschend.

Über die primären, nämlich geruchlichen, Eigenschaften hinaus verfügen die Verbindungen der Formel (I) zusätzlich über positive Sekundäreigenschaften, insbesondere ein gutes Haftungsvermögen und eine hohe Substantivität im Vergleich zu Riechstoffen mit ähnlichen Geruchseigenschaften, sowie eine hohe Stabilität in bestimmten Medien und Zubereitungen, eine hohe Ausgiebigkeit und sind zudem biologisch abbaubar.

Eine erfindungsgemäß einzusetzende Verbindung der Formel (I) weist insbesondere in weitgehend neutralen, insbesondere jedoch in alkalischen und/oder in oxidierenden Medien eine hohe, ganz hervorragende Stabilität auf. Insbesondere wegen dieser Eigenschaften eignen sich die Verbindungen der Formel (I) ausgezeichnet für die Verwendung als Riechstoffe, und zwar insbesondere, wenn sie in parfümierten Artikeln oder Zubereitungen eingesetzt werden, die einen pH-Wert von 5,5 oder größer besitzen, vorzugsweise von größer oder gleich 6, bevorzugt von größer 7, weiter bevorzugt von größer 7,5, insbesondere bevorzugt von größer 8; ferner in oxidierend wirkenden Zubereitungen, die vorzugsweise einen pH-Wert von größer oder gleich 7 aufweisen, bevorzugt in oxidierend wirkenden Zubereitungen mit einem pH-Wert von größer oder gleich 8. Die angegebenen pH-Werte beziehen sich dabei jeweils auf bei 25°C gemessene Werte.

Im Zusammenhang mit der bevorzugten Verwendung zum Vermitteln, Modifizieren und/oder Verstärken einer hierin beschriebenen Geruchsnote steht auch die Erkenntnis, dass die Verbindungen der Formel (I) hervorragend als sogenannte Booster (Verstärker; Enhancer) fungieren können.

Insofern wird hierin auch eine Verwendung bzw. ein Verfahren zum Modifizieren und/oder Verstärken (Boosten) eines Geruchs mit einer, mehreren oder sämtlichen der hierin beschriebenen Noten, vorzugsweise der Note(n) blumig, fruchtig und/oder holzig, bereitgestellt, umfassend folgenden Schritt:
- Vermischen eines oder mehrerer Riechstoffe mit einer, mehreren oder sämtlichen der hierin beschriebenen Noten, vorzugsweise der Note(n) blumig, fruchtig und/oder holzig, mit einer Menge an Verbindung(en) der Formel (I), die ausreicht, um den Geruchseindruck des bzw. der Riechstoffe bzw. die besagte(n) Note(n) sensorisch zu modifizieren und/oder zu verstärken.

Eine erfindungsgemäß zu verwendende Verbindung der Formel (I) kann zudem die Intensität einer Riechstoffmischung (Riechstoffkomposition) verstärken und das Gesamtbild der Mischung geruchlich abrunden. Die hierin beschriebenen Verbindungen können daher eingesetzt werden, um einer Riechstoffkomposition mehr Fülle, Frische, (Strahl-) Kraft, Ausstrahlung, Glanz, Abrundung, Harmonie und/oder Natürlichkeit zu verleihen.

Weiterhin eignen sich Verbindungen der Formel (I) als Mittel zum Erhöhen der Substantivität und/oder Retention einer Riechstoffmischung und/oder als Fixateur.

Die Herstellung einer erfindungsgemäß zu verwendenden Verbindung der Formel (I) kann mittels an sich bekannter Reaktionen und Verfahren erfolgen. Wie dem Fachmann bekannt ist, kann z.B. durch eine Knoevenagel-Reaktion mit Cycanessigsäure und 2-Ethylhexanal, mit entsprechenden Katalysatoren und den entsprechenden Bedingungen, eine Verbindung der Formel (I) hergestellt werden (die dafür eingesetzten Edukte sind kommerziell erhältlich).

Eine erfindungsgemäß einzusetzende Verbindung der Formel (I) wird im Rahmen der erfindungsgemäßen Verwendung üblicherweise in einer sensorisch wirksamen Menge eingesetzt, d.h. in einer Gesamtmenge, in der sie eine sensorische Wirkung entfalten. Vorzugsweise wird die erfindungsgemäß einzusetzende Verbindung der Formel (I) zusammen mit anderen Riechstoffen eingesetzt. Solche Riechstoffkompositionen können in üblicher Weise hergestellt werden, beispielsweise durch einfaches Vermischen oder Homogenisieren der Bestandteile. Bei diesen weiteren Riechstoffen kann es sich um beliebige weitere Riechstoffe handeln.

Ein weiterer Aspekt der vorliegenden Erfindung betrifft daher eine Riechstoffkomposition umfassend oder bestehend aus einer oder mehreren Verbindung(en) der Formel (la) und/oder der Formel (Ib) 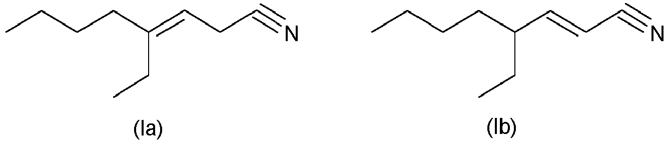 und einem, zwei, drei, vier, fünf, sechs, sieben, acht, neun, zehn oder mehr weiteren Riechstoff(en), wobei in einer erfindungsgemäßen Riechstoffkomposition das Gewichtsverhältnis der Gesamtmenge an Verbindung(en) der Formel (I) zu der Gesamtmenge an weiterem/n Riechstoff(en) im Bereich von 1 : 1000 bis 1 : 0,1, vorzugsweise von 1 : 1000 bis 1: 0,5 liegt.

Hierbei ist es bevorzugt, wenn die Gesamtmenge an Verbindung(en) der Formel (I), bezogen auf das Gesamtgewicht der Riechstoffkomposition, im Bereich von 0,0001 bis 99,9 Gew.-%, vorzugsweise 0,001 bis 99,5 Gew.-%, besonders bevorzugt 0,01 bis 99 Gew.-%, 0,01 bis 90 Gew.-%, 0,05 bis 80 Gew.-%, 0,1 bis 70 Gew.-%, 0,25 bis 50 Gew.-%, 0,5 bis 40 Gew.-% oder 0,75 bis 25 Gew.-%, liegt.

Sofern eine Verbindung der Formel (I) hauptsächlich eingesetzt wird, um einer Riechstoffkomposition mehr (Aus-)Strahlung, Abrundung, Harmonie und/oder Natürlichkeit zu verleihen und/oder bestimmte (durch weitere Riechstoffe bereits vorhandene) Noten zu verstärken, kann die Gesamtmenge der Verbindung(en) der Formel (I) auch vergleichsweise niedrig gewählt werden und z.B. bevorzugt im Bereich von 0,01 bis 5 Gew.-%, weiter bevorzugt im Bereich von 0,1 bis 2 Gew.-%, bezogen auf die Gesamtmenge der Riechstoffkomposition, liegen. Sofern innerhalb der bevorzugten Konzentrationsbereiche eine vergleichsweise niedrige Konzentration gewählt wird, kann es in Abhängigkeit von den weiteren Komponenten der jeweiligen Komposition im Einzelfall noch nicht zu der Vermittlung der hierin angegebenen Eigengeruchsnoten kommen. So kann der Fachmann je nach gewünschtem Effekt einer Verbindung der Formel (I) eine für die jeweilige Anwendung günstige Menge an Verbindung(en) der Formel (I) auswählen.

Eine Verbindung der Formel (I) eignet sich wegen ihrer olfaktorischen Eigenschaften vorzüglich für den Einsatz in erfindungsgemäßen Riechstoffkompositionen. Eine Verbindung der Formel (I) kann vorteilhafterweise mit einer Vielzahl weiterer Riechstoffe kombiniert und in zahlreichen unterschiedlichen Produkten und Artikeln verwendet werden.

Vorzugweise gilt für eine erfindungsgemäße Riechstoffkomposition, dass die Gesamtmenge der Verbindung(en) der Formel (I) in der Riechstoffkomposition in einer sensorisch wirksamen Menge enthalten ist, vorzugsweise in einer Menge, die ausreicht, um eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens einer der Geruchsnoten ausgewählt aus der Gruppe bestehend aus den Noten grün, nitrilig, rosig und Iris- und Karottennoten, bevorzugt rosig mit Iris- und Karottennoten, zu vermitteln und/oder zu verstärken und/oder eine oder mehrere Geruchsnoten eines bzw. des / der weiteren Riechstoffs/e der Riechstoffkomposition in Richtung einer oder mehrerer dieser Geruchsnoten zu modifizieren.

Besonders bevorzugt ist es, wenn der bzw. ein, mehrere oder sämtliche der weiteren Riechstoffe (ebenfalls) eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens eine der Geruchsnoten ausgewählt aus der Gruppe bestehend aus den Noten holzig, fruchtig und blumig, besonders bevorzugt ausgewählt aus der Gruppe bestehend aus den Noten fruchtig und blumig, vermitteln, modifizieren und/oder verstärken. Entsprechend einer alternativen Ausgestaltung kann bzw. können der bzw. ein, mehrere oder sämtliche der weiteren Riechstoffe aber auch eine oder mehrere andere bzw. unterschiedliche Geruchsnoten vermitteln, modifizieren und/oder verstärken.

Beispiele für Riechstoffe, die im Rahmen der vorliegenden Erfindung vorteilhafterweise mit einer Verbindung der Formel (I) kombiniert werden können, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001.

Im Einzelnen seien genannt: Extrakte aus natürlichen Rohstoffen wie ätherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-ÖI; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernnadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-ÖI; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierte Inhaltsstoffe;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;
der acyclischen Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Citronellal; 7-Methoxy-3,7-dimethyloctanal; 2,6,10-Trimethyl-9-undecenal; Geranylaceton; sowie die Dimethyl- und Diethylacetale von Geranial, Neral,
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-lonon; beta-Ionon; alpha-n-Methylionon; beta-n-Methylionon; alpha-Isomethylionon; beta-Isomethyl-ionon; alpha-Iron; beta-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methano-naphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; 1,1-Dimethoxycyclododecan; (Ethoxymethoxy)cyclo-dodecan; alpha-Cedrenepoxid; 3a,6,6,9a-Tetramethyldodecahydro-naphtho[2,1-b]furan; 3a-Ethyl-6,6,9a-trimethyldodecahydronaphtho[2,1b]furan; 1,5,9-Tri-methyl-13-oxabicyclo[10.1,0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1-Ethoxyvinyl)-3,3,5,5-tetramethylcyclohexanon; 4-tert.-Pentylcyclo-hexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbald ehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentyl-cyclo-hexyl-acetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5, bzw. 6-inden-ylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der araliphatischen Alkohole wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenyl-propanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha, alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethyl-isoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetra-hydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-tert.-Butylphenyl)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenz-aldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylaceto-phenon; 4-Phenyl-2-butanon; 4-(4-Hydroxyphenyl)-2-butanon; 1-(2-Naphtha-lenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-di-methyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-aceto-naphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butyl-phenyl)propanal oder 2,4-Dimethyl-3-cyclohexencarbaldehyd; 6-Isopropyl-chinolin; 6-Isobutylchinolin; 6-sec.-Butylchinolin; 2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; 1,16-Hexadeca-nolid; 9-Hexadecen-1,16-olid; 10-Oxa-1,16-hexadecanolid; 11-Oxa-1,16-hexa-decanolid; 12-Oxa-1,16-hexadecanolid; Ethylen-1,12-dodecandioat; Ethylen-1,13-tridecandioat; 2,3-Dihydrocumarin; Octahydrocumarin.

Besonders bevorzugt sind solche Riechstoffe, die eine blumige und/oder fruchtige Geruchsnote vermitteln. Insbesondere bei solchen Riechstoffen wird durch eine erfindungsgemäße zu verwendende Verbindung der Formel (I) vorteilhafterweise (zumindest teilweise) eine geruchliche Verstärkung der blumigen und/oder fruchtigen Noten erreicht.

Blumige Riechstoffe, mit denen erfindungsgemäß zu verwendende Verbindungen der Formel (I) (insbesondere in erfindungsgemäßen Riechstoffkompositionen) besonders vorteilhaft kombiniert werden können, sind vorzugsweise ausgewählt aus der Gruppe bestehend aus:
Hydroxycitronellal, Methoxycitronellal, Cyclamenaldehyd [2-Methyl-3-(4-isopropylphenyl)propanal], 1-(4-Isopropyl-cyclohexyl)ethanol (Mugetanol®), 4-tert.-Butyl-α-methyldihydrozimtaldehyd (Lilial®), cis-Hexahydrocuminylalkohol (Mayol®), 3-[4-(1,1-Dimethylethyl)phenyl]propanal (Bourgeonal®), 2,2-Dimethyl-3-(3-methylphenyl)propanol (Majantol®), 3-Methyl-3-(3-methylbenzyl)-butan-2-ol, 2-Isobutyl-4-methyltetrahydro-2H-pyran-4-ol (Florosa®), 2-Methyl-3-(3,4-methylendioxyphenyl)propanal (Heliofolal®), 4-(4-Hydroxy-4-methylpentyl)-3-cyclohexencarbaldehyd (Lyral®), 4-(Octahydro-4,7-methano-5H-inden-5-yliden-butanal (Dupical®), Vernaldehyd, 4-(4-Methyl-3-penten-1-yl)-3-cyclohexencarbaldehyd (Vertomugal®), Octahydro-5-(4-methoxybutyliden)-4,7-methano-1H-inden (Mugoflor®). 2,6-Dimethyl-2-heptanol (Freesiol®), 1-Ethyl-1-methyl-3-phenylpropanol (Phemec®), 2,2-Dimethyl-3-phenyl-1-propanol (Muguet alcohol), Profarnesol, Dihydrofarnesol, Farnesol, Nerolidol, Hydroxycitronellaldimethylacetal, Hexylbenzoat, Geraniol, Nerol, Linalool, Tetrahydrogeraniol, Tetrahydrolinalool, Ethyllinalool, Geranyltiglinat, Phenethylalkohol (2-Phenylethylalkohol), Citronellol, Rosenoxid, 2-Methyl-5-phenylpentanol (Rosaphen), 3-Methyl-5-phenylpentanol (Phenoxanol), Methyldihydrojasmonat (Hedion®, Hedione® high cis), 2-Heptylcyclopentanon (Projasmon P), cis-Jasmon, Dihydrojasmon, Zimtalkohol (3-Phenyl-2-propen-1-ol), Dihydrozimtalkohol (3-Phenylpropanol), 2-Methyl-4-phenyl-1,3-dioxolan (Jacinthaflor®) und Dihydromyrcenol (2,6-Dimethyl-7-octen-2-ol).

Fruchtige Riechstoffe, mit denen erfindungsgemäße zu verwendende Verbindungen der Formel (I) vorteilhaft kombiniert werden können, und die demnach besonders bevorzugte (weitere) Riechstoffe einer erfindungsgemäßen Riechstoffkompositionen sind, sind vorzugsweise ausgewählt der Gruppe bestehend aus:
2-Methyl-buttersäureethylester, 4-(p-Hydroxyphenyl)-2-butanon, Ethyl-3-methyl-3-phenylglycidat, Buttersäureisoamylester, Essigsäureisoamylester, Essigsäure-n-butylester, Buttersäureethylester, 3-Methyl-buttersäureethylester, n-Hexansäureethylester, n-Hexansäureallylester, Ethyl-2-trans-4-cis-decadienoat, 1,1-Dimethoxy-2,2,5-trimethyl-4-hexan, 2,6-Dimethyl-5-hepten-1-al, gamma-Undecalacton, gamma-Nonalacton, Hexanal, 3Z-Hexenal, n-Decanal, n-Dodecanal, Citral, Vanillin, Ethylvanillin, Maltol, Ethylmaltol und deren Mischungen.

Erfindungsgemäße Riechstoffkompositionen, welche eine Verbindung der Formel (I) enthalten, können in flüssiger Form, unverdünnt oder mit einem Lösungsmittel verdünnt sein bzw. werden und vorteilhafterweise zur Parfümierung eingesetzt werden. Bevorzugte Lösungsmittel hierfür sind Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat, Triacetin und Diacetin.

Des Weiteren können erfindungsgemäße Riechstoffkompositionen an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulosebasierende Stoffe, Zucker, Dextrine (z.B. Maltodextrin) oder Kunststoffe wie PVC, Polyvinylacetate oder Polyurethane sein. Die resultierende Kombination aus erfindungsgemäßer Komposition und Trägerstoff ist ebenfalls als erfindungsgemäße Riechstoffkomposition zu verstehen oder kann als erfindungsgemäßer Artikel (wie hierin weiter unten beschrieben) vorliegen.

Erfindungsgemäße Riechstoffkompositionen oder Artikel (wie hierin weiter unten beschrieben) können auch mikroverkapselt, sprühgetrocknet, als Einschluss-Komplexe oder als Extrusions-Produkte vorliegen und - im Falle einer Riechstoffkomposition - in dieser Form z.B. einem zu parfümierenden Artikel hinzugefügt werden (wie hierin weiter unten beschrieben).

Gegebenenfalls können die Eigenschaften der derart modifizierten Kompositionen oder Artikel durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Artikel dar.

Eine Mikroverkapselung kann beispielsweise durch das sogenannte Koazervationsverfahren mit Hilfe von Kapselmaterialien z.B. aus polyurethanartigen Stoffen oder Weichgelatine, erfolgen.

Sprühgetrocknete Produkte werden vorzugsweise durch Sprühtrocknung einer die Riechstoffkomposition enthaltenden Emulsion, bzw. Dispersion hergestellt, wobei als Trägerstoffe modifizierte Stärken, Proteine, Dextrin und pflanzliche Gummen verwendet werden können.

Einschluss-Komplexe können z.B. durch Eintragen von Dispersionen von der Riechstoffkomposition und Cyclodextrinen oder Harnstoffderivaten in ein geeignetes Lösungsmittel, z.B. Wasser, hergestellt werden.

Extrusions-Produkte können z.B. durch Verschmelzen der Riechstoffkompositionen mit einem geeigneten wachsartigen Stoff und durch Extrusion mit nachfolgender Erstarrung, gegebenenfalls in einem geeigneten Lösungsmittel, z.B. Isopropanol, erhalten werden.

Ein weiterer Aspekt der vorliegenden Erfindung betrifft die Verwendung einer erfindungsgemäßen Riechstoffkomposition zum Vermitteln, Modifizieren und/oder Verstärken einer oder mehrerer Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens einer der Geruchsnoten ausgewählt aus der Gruppe bestehend aus den Noten grün, nitrilig, rosig und Iris- und Karottennoten, bevorzugt rosig mit Iris- und Karottennoten.

Für bevorzugte Ausgestaltungen gilt hierbei das oben im Zusammenhang mit einer erfindungsgemäßen Verwendung oder erfindungsgemäßen Riechstoffkomposition Gesagte entsprechend.

Erfindungsgemäße Riechstoffkompositionen können vorteilhafterweise in konzentrierter Form, in Lösungen oder in oben beschriebener modifizierter Form für die Herstellung von erfindungsgemäßen parfümierten Artikeln verwendet werden, z. B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-ÖI-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Ein weiterer Aspekt der vorliegenden Erfindung betrifft dementsprechend einen parfümierten Artikel, umfassend oder bestehend aus
(i) einer erfindungsgemäßen Riechstoffkomposition (wie hierin beschrieben), und
(ii) einem oder mehreren weiteren Bestandteil(en), vorzugsweise wenigstens einem oder mehreren, vorzugsweise einem, zwei, drei, vier, fünf oder mehr, Zusatz-, Hilfs- und/oder Wirkstoff(en).

Für bevorzugte Ausgestaltungen gilt hierbei das oben im Zusammenhang mit erfindungsgemäßen Verwendungen oder erfindungsgemäßen Riechstoffkompositionen Gesagte entsprechend.

Bevorzugt ist ein erfindungsgemäßer Artikel ausgewählt aus der Gruppe bestehend aus Wasch- und Reinigungsmitteln, Hygiene- oder Pflegeprodukten, vorzugsweise im Bereich der Körper- und Haarpflege, der Kosmetik und des Haushalts, vorzugsweise aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -Iotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

Außerdem gilt für einen erfindungsgemäßen parfümierten Artikel vorzugsweise, dass Bestandteil (i) in einer sensorisch wirksamen Menge enthalten ist, vorzugsweise in einer Menge, die ausreicht, dass ein Verbraucher eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens eine der Geruchsnoten ausgewählt aus der Gruppe bestehend aus den Noten grün, nitrilig, rosig und Iris- und Karottennoten, bevorzugt rosig mit Iris-und Karottennoten, wahrnimmt.

Bevorzugt ist auch, wenn der parfümierte Artikel eine Gesamtmenge an Verbindung(en) der Formel (I), bezogen auf das Gesamtgewicht des Artikels, im Bereich von 0,00001 bis 10 Gew.-%, vorzugsweise 0,0001 bis 5 Gew.-%, besonders bevorzugt 0,001 bis 2 Gew.-%, weiter bevorzugt 0,005 bis 1 Gew.-%, enthält.

Die oben beschriebenen Zusatz-, Hilfs- und/oder Wirkstoffe sind vorzugsweise keine Riechstoffe und sind, falls enthalten, vorzugsweise ausgewählt aus der Gruppe bestehend aus:
Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildner, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, (weitere) Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-betadicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, fette Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Farbstoffe und farbschützende Mittel sowie Pigmente, vorzugsweise die in WO 2005/123101 genannten, Antikorrosiva, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, haarwachstumsmodulierende Mittel (haarwachstumsfördend oder haarwachstumshemmend), vorzugsweise die in EP 2168570 und EP 2193785 genannten oder Silikone und Silikonderivate, vorzugsweise die in WO 2008/046676 genannten,
bevorzugt aus der Gruppe bestehend aus Konservierungsmitteln, anorganischen Salzen, Chelatbildnern, Tensiden, haut- und/oder haarpflegenden Mitteln, Enzymen Emulgatoren, Fetten, fetten Ölen, Wachsen, Fettalkoholen, Silikonen, Silikonderivaten und Wasser.

Ein weiterer Aspekt der vorliegenden Erfindung betrifft ein Verfahren zum Parfümieren eines Artikels (für bevorzugte Artikel s. oben), umfassend oder bestehend aus folgenden Schritten:
(a) Bereitstellen
   (a.1) einer erfindungsgemäßen Riechstoffkomposition (wie hierin beschrieben),
      oder
   (a.2) einer oder mehreren Verbindung(en) der Formel (la) und/oder der Formel (Ib) 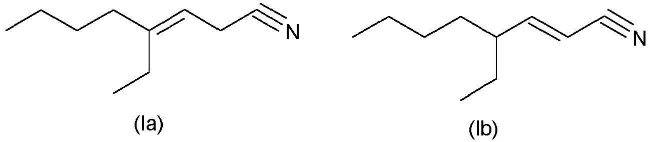 und optional einem, zwei, drei, vier, fünf, sechs, sieben, acht, neun, zehn oder mehr weiterem/n Riechstoff(en), wobei das Gewichtsverhältnis der Gesamtmenge an Verbindung(en) der Formel (I) zu der Gesamtmenge an weiterem/n Riechstoff(en) im Bereich von 1 : 1000 bis 1 : 0,1, vorzugsweise von 1 : 1000 bis 1: 0,5 liegt,
      und
(b) Hinzufügen der Riechstoffkomposition (a.1) bzw. Verbindungen(en) / Riechstoffen (a.2) zu dem zu parfümierenden Artikel, in einer sensorisch wirksamen Menge, vorzugsweise in einer Menge, die ausreicht, um eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens eine der Geruchsnoten ausgewählt aus der Gruppe bestehend aus den Noten grün, nitrilig, rosig und Iris- und Karottennoten, bevorzugt rosig mit Iris- und Karottennoten, zu vermitteln, zu verstärken und/oder zu modifizieren.

Weiterhin betrifft die vorliegende Erfindung ein Verfahren zum Parfümieren von Haaren, Haut, textilen Fasern, Oberflächen und/oder Raumluft umfassend oder bestehend aus folgenden Schritten:
(a) Bereitstellen
   (a.1) einer erfindungsgemäßen Riechstoffkomposition (wie hierin beschrieben), vorzugsweise enthaltend ein Tensid oder eine Tensidmischung,
      oder
   (a.2) einer oder mehreren Verbindung(en) der Formel (la) und/oder der Formel (Ib) 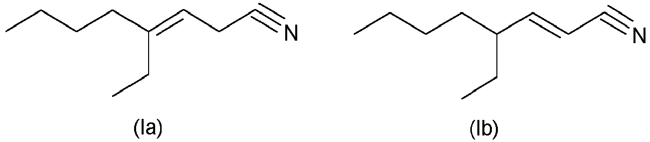 und optional einem, zwei, drei, vier, fünf, sechs, sieben, acht, neun, zehn oder mehr weiterem/n Riechstoff(en), wobei das Gewichtsverhältnis der Gesamtmenge an Verbindung(en) der Formel (I) zu der Gesamtmenge an weiterem/n Riechstoff(en) im Bereich von 1 : 1000 bis 1 : 0,1, vorzugsweise von 1 : 1000 bis 1: 0,5 liegt,
      und zudem bevorzugt einem Tensid oder einer Tensidmischung, oder
   (a.3) einem erfindungsgemäßen Artikel (wie hierin beschrieben), vorzugsweise enthaltend ein Tensid oder eine Tensidmischung,
      und
(b) Auftragen bzw. Einbringen der Riechstoffkomposition (a.1) bzw. der Verbindungen(en) / Riechstoffe (a.2) bzw. des Artikels (a.3) auf die zu parfümierende(n) Haare bzw. Haut bzw. Fasern bzw. Oberfläche bzw. in die zu parfümierende Raumluft in einer sensorisch wirksamen Menge, vorzugsweise in einer Menge, die ausreicht, dass der Verbraucher eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens eine der Geruchsnoten ausgewählt aus der Gruppe bestehend aus den Noten grün, nitrilig, rosig und Iris- und Karottennoten, bevorzugt rosig mit Iris- und Karottennoten, wahrnimmt.

Für bevorzugte Ausgestaltungen dieser Verfahren gilt wiederum das oben im Zusammenhang mit erfindungsgemäßen Verwendungen oder erfindungsgemäßen Riechstoffkompositionen oder erfindungsgemäßen Artikeln Gesagte entsprechend.

Nachfolgend wird die Erfindung anhand von ausgewählten Beispielen näher erläutert. Sofern nicht anders angegeben, beziehen sich alle Angaben auf das Gewicht.
Verwendete Abkürzungen: DPG = Dipropylenglycol, TEC = Triethylcitrat

### Beispiel 1: Herstellung von 4-Ethyl-octen-2/3-nitril

In einen 1000 ml Rührwerk mit Wasserabscheider wurden 254 g 2-Ethyhexanal, 38 g Ammoniumacetat und 46 g Essigsäure in 520 g Toluol vorgelegt. Danach unter Rühren portionsweise mit 189 g Cyanessigsäure bei RT versetzt. Anschließend 14 h am Wasserabscheider refluxiert und nach dem Abkühlen mit 10%iger Schwefelsäure, 5%iger Natronlauge bzw. Wasser waschen. Die organische Phase wurde eingeengt und das Rohprodukt (280 g) an einer 40 cm Vigreux-Kolonne im Vakuum fraktioniert destilliert.
Ausbeute: 190 g (63,9% d. Th.) Sdp.: 80°-85°C/0,8 mbar
GC-Auswertung (20m DB-WAX, Innendurchmesser 0,18µm / 60-9-220°C Kaltaufgabesystem)
Das Produkt besteht aus 4 Isomeren, 4,5% (E/Z) 4-Ethyl-octen-2-nitril und 95,5% (E/Z) 4-Ethyl-octen-3-nitril
(E/Z) 4-Ethyl-octen-2-nitril
MS: m/z (%) = 150(2), 136(7), 122(14), 110(38), 97(54), 80(100), 70(48), 55(78), 41(89)
MS: m/z (%) = 150(2), 136(5), 122(12), 110(27), 97(51), 80(67), 70(65), 56(100), 41(94)
(E/Z) 4-Ethyl-octen-3-nitril
MS: m/z (%) = 151(2), 136(2), 122(4), 110(26), 94(32), 81(23), 69(100), 55(21), 41(43)
MS: m/z (%) = 151(2), 136(2), 122(5), 110(25), 94(32), 81(22), 69(100), 55(21), 41(42)

### Beispiel 1a: Herstellung von 4-Ethyl-octen-2/3-nitril

In einen 1000 ml Rührwerk mit Wasserabscheider und Tropftrichter wurden 254 g 2-Ethyhexanal und 189 g Cyanessigsäure in 500 ml Cyclohexan vorgelegt. Danach unter Rühren und Sieden 65 g 3-Picolin zudosiert. Anschließend 26 h am Wasserabscheider refluxiert und nach dem Abkühlen mit 10%iger Schwefelsäure, 5%iger Natronlauge bzw. Wasser waschen. Die organische Phase wurde eingeengt und das Rohprodukt (250 g) erhalten. Das Rohprodukt besteht aus 4 Isomeren, 16,5% (E/Z) 4-Ethyl-octen-2-nitril und 63,9% (E/Z) 4-Ethyl-octen-3-nitril

Die Rohware wird an einer Spaltrohr-Kolonne im Vakuum fraktioniert destilliert.

Ausbeute an Produkt: 37,5 g (E/Z) 4-Ethyl-octen-2-nitril (95,4%ig)
Sdp.: 75°-80°C/0,8 mbar
GC-Auswertung (20m DB-WAX, Innendurchmesser 0,18µm / 60-9-220°C Kaltaufgabesystem)

### Beispiel 2: Parfümkomposition (Riechstoffkomposition)

| | |
|---|---|
| AMAROCIT® 10% DPG | 6 |
| AMBRETTOLIDE | 2 |
| AMBRINOL S 10% DPG | 6 |
| BENZOIN OLIFFAC TYPE BASE | 8 |
| CASSIS 345B TYPE BASE W/O MYRCENE | 10 |
| DECALACTON DELTA 10% DPG | 3 |
| DIHYDROIONON BETA | 6 |
| DIHYDROJASMON 10% DPG | 3,5 |
| DIHYDROMYRCENOL | 6 |
| DIPROPYLENGLYCOL | 63 |
| EBANOL 10% DPG | 6 |
| ETHYLENBRASSYLAT | 80 |
| ETHYLLINALOOL | 10 |
| ETHYLMALTOL 10% DPG | 1 |
| FLOROSA BM / PYRANOL | 5 |
| GLOBALIDE® | 18 |
| GLOBANONE® | 12 |
| HEDION | 140 |
| HELIONAL | 10 |
| HELIOTROPIN/PIPERONAL | 3 |
| HEXYLACETAT | 3 |
| IONON ALPHA | 30 |
| IRISNITRIL 1% DPG | 4 |
| IRON ALPHA 10% DPG | 3 |
| ISO E SUPER | 80 |
| KOAVONE | 6 |
| LACTOJASMONE | 1 |
| MACROLIDE® SUPRA | 10 |
| MENTHANYLACETAT | 10 |
| METHYLHEPTENON-6,5,2 10% DPG | 2 |
| MUSCENONE | 1 |
| MYSORE ACETAT | 15 |
| OCTAHYDROCUMARIN | 1,5 |
| ORANGENOEL 5X | 1 |
| PENTYLACETAT N 1% DPG | 10 |
| PHENOXANOL | 25 |
| SANDALORE | 15 |
| SANDRANOL® | 20 |
| TERPINYLACETAT | 20 |
| TETRAHYDROLINALOOL | 30 |
| VANILLIN | 8 |
| YSAMBER® K | 6 |
| DIPROPYLENGLYKOL | 300 |

Nach Meinung der Parfümeure wird diese Parfümkomposition durch den Zusatz von 0,5 Gew.-% einer Verbindung der Formel (la) oder (Ib) bzw. einer Mischung aus (la) und (Ib) trockener, pudriger, abgerundeter und harmonischer, wobei eine deutliche Iris-Veilchen-und Rosennote hinzukommt und die holzigen und blumigen Aspekte verstärkt werden. Die erfindungsgemäße Kombination bzw. Verwendung verleiht der Komposition einen eigenen Charakter und verbindet die unterschiedlichen geruchlichen Elemente.

### Beispiel 3: Parfümkomposition (Riechstoffkomposition)

| | |
|---|---|
| AMBERWOOD® F | 30 |
| AMBRA CORE | 70 |
| AMBROCENIDE® 10 DPG 10% DPG | 10 |
| AMBROXIDE | 10 |
| AMYLSALICYLAT N | 3 |
| BERGAMOTT ECO ESSENCE | 15 |
| CARDAMOMENOEL GUATEMALA | 2 |
| CEDERNHOLZOEL VIRGINIA | 10 |
| CHOCOLAT NOIR 0.1% DPG | 10 |
| CUMARIN | 10 |
| DAMASCENON 10% DPG | 5 |
| DIPROPYLENGLYCOL | 449 |
| GLOBALIDE® | 100 |
| HEDION | 20 |
| HELIOTROPIN/PIPERONAL | 3 |
| HEXENYLSALICYLAT CIS-3 | 8 |
| IONON BETA | 3 |
| ISO E SUPER NON DISCOLORING | 200 |
| LINALOOL | 5 |
| RED BERRIES EXTRACT | 2 |
| SANDALORE | 5 |
| SANDRANOL® | 2 |
| TIMBEROL® | 10 |
| VANILLIN | 10 |
| DIPROPYLENGLYCOL | 8 |

Nach Meinung der Parfümeure erwacht diese Parfümkomposition durch den Zusatz von 2 Gew.-% einer Verbindung der Formel (la) oder (Ib) bzw. einer Mischung aus (la) und (Ib) 10%ig in DPG. Der Eindruck von Blumigkeit wird verstärkt. Die Komposition wirkt holziger, abgerundeter und harmonischer, wobei eine natürliche Note hinzukommt.

### Beispiel 4: Parfümkomposition (Riechstoffkomposition)

| | |
|---|---|
| ACETYL PYRAZINE-2 / 1% IN TEC 1% DPG | 4 |
| AMBROXIDE | 15 |
| BOISIRIS | 60 |
| CEDREN | 5 |
| CETONE ALPHA | 5 |
| DIPROPYLENGLYCOL | 25 |
| ETHYLVANILLIN 10% DPG | 5 |
| GLOBALIDE® | 45 |
| HEDION HC/70 | 300 |
| IONON BETA | 15 |
| IONON BETA NAT. | 15 |
| IRISNITRIL 0.1% DPG | 20 |
| ISO E SUPER | 100 |
| ISORALDEIN 70 | 35 |
| LAIT CHAUD | 1 |
| NEROLIDOL | 30 |
| NONENALTRANS-2 0.1% DPG | 5 |
| VELVIONE | 15 |

Nach Meinung der Parfümeure gewinnt die Parfümkomposition durch den Zusatz von 5 Gew.-% einer Verbindung der Formel (la) oder (Ib) bzw. einer Mischung aus (la) und (Ib) 1%ig in DPG. Die blumigen Aspekte werden verstärkt. Die Komposition wirkt holziger, abgerundeter und harmonischer, wobei eine natürliche Note hinzukommt.

### Beispiel 5: Shampoo

Das erfindungsgemäß zu verwendende Produkt aus Beispiel 1 bzw. 1a wurde in einer Dosierung von 0,5 Gew.-% in eine Shampoo-Grundmasse folgender Zusammensetzung eingearbeitet:

| | |
|---|---|
| Natriumlaurylethersulfat | 12% |
| (z.B. Texapon NSO, Fa. Cognis Deutschland GmbH) Cocamidopropylbetain | 2% |
| (z.B. Dehyton K, Fa. Cognis Deutschland GmbH) Natriumchlorid | 1,4% |
| Citronensäure | 1,3% |
| Phenoxyethanol, Methyl-, Ethyl-, Butyl-, und Propylparaben | 0,5% |
| Wasser | 82,8% |

Der pH-Wert der Shampoo-Grundmasse lag bei etwa 6. Hieraus wurden 100 mL einer 20 Gew.%-igen wässrigen Shampoo-Lösung hergestellt. In dieser Shampoo-Lösung wurden 2 Haarsträhnchen gemeinsam für 2 Minuten gewaschen und anschließend 20 Sekunden unter fließendem handwarmem Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt.
Geruchsbeschreibung beider Haarsträhnen: stark grün, nitrilig, rosig mit Iris- und Karottennoten

### Beispiel 6: Weichspüler

Die Parfümkomposition aus Beispiel 2 (nach Zusatz von 0,5 Gew.-% des Produktes aus Beispiel 1 bzw. 1a) wurde in einer Dosierung von 0,5 Gew.-% in eine Weichspüler-Grundmasse folgender Zusammensetzung eingearbeitet:

| | |
|---|---|
| Quarternäres Ammoniummethosulfat (Esterquat), ca. 90% | 5,5% |
| (z.B. Rewoquat WE 18, Fa. Witco Surfactants GmbH) Alkyldimethylbenzylammoniumchlorid, ca. 50% | 0,2% |
| (z.B. Preventol R50, Fa. Bayer AG) Farblösung, ca. 1%-ig | 0,3% |
| Wasser | 94,0% |

Der pH-Wert der Weichspüler-Grundmasse lag im Bereich von 2 bis 3. Zwei Stofflappen wurden mit 370 g einer 1%-igen wässrigen Weichspüler-Lösung auf Basis der 0,5% Gew.-% der Parfümkomposition aus Beispiel 2 umfassenden Weichspüler-Grundmasse in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweisst, und einer zum Trocknen aufgehängt. Anschließend wurden beide Lappen durch ein Panel geruchlich beurteilt.

Geruchsbeschreibung beider Stofflappen: trockener, pudriger, abgerundeter und harmonische, mit einer deutlichen Iris-Veilchen- und Rosennote.

### Beispiel 7: Waschpulver

Die Parfümölkomposition aus Beispiel 3 (nach Zusatz von 0,5 Gew.-% des Produkts aus Beispiel 1 bzw. 1a, 10%ig in DPG) wurde in einer Dosierung von 0,4 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife Entschäumer DOW CORNING 2-4248S | 3,2 % |
| POWDERED ANTIFOAM, Silikonöl auf Zeolith als Trägermaterial | 3,9 % |
| Zeolith 4A | 28,3 % |
| Na-Carbonat | 11,6 % |
| Na-Salz eines Copolymers aus Acryl- und Maleinsäure (Sokalan CP5) | 2,4 % |
| Na-Silikat | 3,0 % |
| Carboxymethylcellulose | 1,2 % |
| Dequest 2066 | 2,8 % |
| ([[(Phosphonomethyl)imino]bis[(ethylennitrilo)bis (methylen)]]tetrakis-phosphonsäure, Natriumsalz) Optischer Aufheller | 0,2 % |
| Na-Sulfat | 6,5 % |
| Protease | 0,4 % |
| Natriumperborat-tetrahydrat | 22,0 % |
| TAED | 1,0 % |

Zwei Stofflappen wurden mit 370 g einer 1%-igen wässrigen Waschpulverlauge auf Basis des 0,4 Gew.-% der Parfümölkomposition aus Beispiel 3 umfassenden Waschpulver-Grundmasse (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweisst, und einer zum Trocknen aufgehängt. Anschließend wurden beide Lappen durch ein Panel geruchlich beurteilt.

Geruchsbeschreibung jeweils: eine schöne blumige Note mit holzigem Unterton ist wahrzunehmen. Die Parfümkomposition wirkt abgerundet, harmonisch, mit einer natürlichen Note.

## Patentansprüche

1. Verwendung einer oder mehrerer Verbindung(en) der Formel (la) und/oder der Formel (Ib), 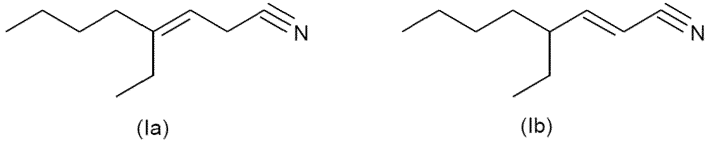 als Riechstoff.

2. Verwendung nach Anspruch 1 zum Vermitteln, Modifizieren und/oder Verstärken einer oder mehrerer Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens einer der Geruchsnoten ausgewählt aus der Gruppe bestehend aus den Noten grün, nitrilig, rosig und Iris- und Karottennoten, bevorzugt rosig mit Iris- und Karottennoten.

3. Riechstoffkomposition umfassend oder bestehend aus einer oder mehreren Verbindung(en) der Formel (la) und/oder der Formel (Ib) 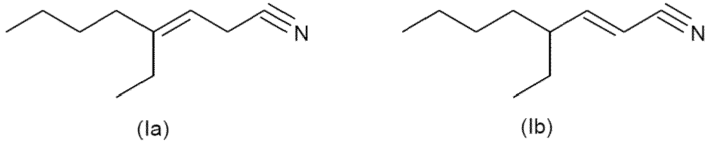 und einem, zwei, drei, vier, fünf, sechs, sieben, acht, neun, zehn oder mehr weiteren Riechstoff(en), wobei das Gewichtsverhältnis der Gesamtmenge an Verbindung(en) der Formel (I) zu der Gesamtmenge an weiterem/n Riechstoff(en) im Bereich von 1 : 1000 bis 1 : 0,1, vorzugsweise von 1 : 1000 bis 1: 0,5 liegt.

4. Riechstoffkomposition nach Anspruch 3, wobei die Gesamtmenge an Verbindung(en) der Formel (I), bezogen auf das Gesamtgewicht der Riechstoffkomposition, im Bereich von 0,0001 bis 99,9 Gew.-%, vorzugsweise 0,001 bis 99,5 Gew.-%, besonders bevorzugt 0,01 bis 99 Gew.-%, liegt.

5. Riechstoffkomposition nach Anspruch 3 oder 4, wobei die Gesamtmenge der Verbindung(en) der Formel (I) in der Riechstoffkomposition in einer sensorisch wirksamen Menge enthalten ist, vorzugsweise in einer Menge, die ausreicht, um eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens einer der Geruchsnoten ausgewählt aus der Gruppe bestehend aus den Noten grün, nitrilig, rosig und Iris- und Karottennoten, bevorzugt rosig mit Iris- und Karottennoten, zu vermitteln und/oder zu verstärken und/oder eine oder mehrere Geruchsnoten eines bzw. des / der weiteren Riechstoffs/e der Riechstoffkomposition in Richtung einer oder mehrerer dieser Geruchsnoten zu modifizieren.

6. Riechstoffkomposition nach einem der Ansprüche 3 bis 5, wobei der bzw. ein, mehrere oder sämtliche der weiteren Riechstoffe (ebenfalls) eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens eine der Geruchsnoten ausgewählt aus der Gruppe bestehend aus den Noten holzig, fruchtig und blumig, besonders bevorzugt ausgewählt aus der Gruppe bestehend aus den Noten fruchtig und blumig, vermitteln, modifizieren und/oder verstärken.

7. Verwendung einer Riechstoffkomposition nach einem der Ansprüche 3 bis 6 zum Vermitteln, Modifizieren und/oder Verstärken einer oder mehrerer Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens einer der Geruchsnoten ausgewählt aus der Gruppe bestehend aus den Noten grün, nitrilig, rosig und Iris- und Karottennoten, bevorzugt rosig mit Iris- und Karottennoten.

8. Parfümierter Artikel, umfassend oder bestehend aus
(i) einer Riechstoffkomposition nach einem der Ansprüche 3 bis 6,
und
(ii) einem oder mehreren weiteren Bestandteil(en), vorzugsweise wenigstens einem oder mehreren, vorzugsweise einem, zwei, drei, vier, fünf oder mehr, Zusatz-, Hilfs- und/oder Wirkstoff(en).

9. Parfümierter Artikel nach Anspruch 8, wobei der Artikel ausgewählt ist aus der Gruppe bestehend aus Wasch- und Reinigungsmitteln, Hygiene- oder Pflegeprodukten, vorzugsweise im Bereich der Körper- und Haarpflege, der Kosmetik und des Haushalts, vorzugsweise aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstüchern, sauren, alkalischen oder neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und - lotionen, After-shave-Cremes und -lotionen, Bräunungscremes und -lotionen, Haarpflegeprodukten, Deodorantien, Antiperspirantien, Produkte der dekorativen Kosmetik, Kerzen, Lampenölen, Räucherstäbchen, Insektiziden, Repellentien und Treibstoffen.

10. Parfümierter Artikel nach Anspruch 8 oder 9, wobei Bestandteil (i) in einer sensorisch wirksamen Menge enthalten ist, vorzugsweise in einer Menge, die ausreicht, dass ein Verbraucher eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens eine der Geruchsnoten ausgewählt aus der Gruppe bestehend aus den Noten grün, nitrilig, rosig und Iris- und Karottennoten, bevorzugt rosig mit Iris- und Karottennoten, wahrnimmt.

11. Parfümierter Artikel nach einem der Ansprüche 8 bis 10, wobei die Gesamtmenge an Verbindung(en) der Formel (I), bezogen auf das Gesamtgewicht des Artikels, im Bereich von 0,00001 bis 10 Gew.-%, vorzugsweise 0,0001 bis 5 Gew.-%, besonders bevorzugt 0,001 bis 2 Gew.-%, weiter bevorzugt 0,005 bis 1 Gew.-%, liegt.

12. Verfahren zum Parfümieren eines Artikels, umfassend oder bestehend aus folgenden Schritten:
(a) Bereitstellen
(a.1) einer Riechstoffkomposition nach einem der Ansprüche 3 bis 6,
oder
(a.2) einer oder mehreren Verbindung(en) der Formel (la) und/oder der Formel (Ib) 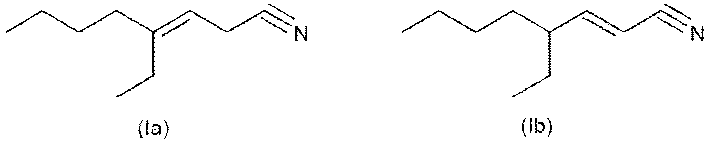 und optional einem, zwei, drei, vier, fünf, sechs, sieben, acht, neun, zehn oder mehr weiterem/n Riechstoff(en), wobei das Gewichtsverhältnis der Gesamtmenge an Verbindung(en) der Formel (I) zu der Gesamtmenge an weiterem/n Riechstoff(en) im Bereich von 1 : 1000 bis 1 : 0,1, vorzugsweise von 1 : 1000 bis 1: 0,5 liegt,
und
(b) Hinzufügen der Riechstoffkomposition (a.1) bzw. Verbindungen(en) / Riechstoffen (a.2) zu dem zu parfümierenden Artikel, in einer sensorisch wirksamen Menge, vorzugsweise in einer Menge, die ausreicht, um eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens eine der Geruchsnoten ausgewählt aus der Gruppe bestehend aus den Noten grün, nitrilig, rosig und Iris- und Karottennoten, bevorzugt rosig mit Iris- und Karottennoten, zu vermitteln, zu verstärken und/oder zu modifizieren.

13. Verfahren zum Parfümieren von Haaren, Haut, textilen Fasern, Oberflächen und/oder Raumluft umfassend oder bestehend aus folgenden Schritten:
(a) Bereitstellen
(a.1) einer Riechstoffkomposition nach einem der Ansprüche 3 bis 6, vorzugsweise enthaltend ein Tensid oder eine Tensidmischung,
oder
(a.2) einer oder mehreren Verbindung(en) der Formel (la) und/oder der Formel (Ib) 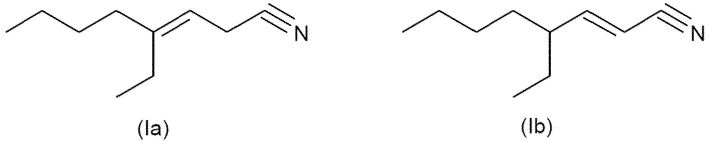 und optional einem, zwei, drei, vier, fünf, sechs, sieben, acht, neun, zehn oder mehr weiterem/n Riechstoff(en), wobei das Gewichtsverhältnis der Gesamtmenge an Verbindung(en) der Formel (I) zu der Gesamtmenge an weiterem/n Riechstoff(en) im Bereich von 1 : 1000 bis 1 : 0,1, vorzugsweise von 1 : 1000 bis 1: 0,5 liegt,
und zudem bevorzugt einem Tensid oder einer Tensidmischung,
oder
(a.3) einem Artikel nach einem der Ansprüche 8 bis 11, vorzugsweise enthaltend ein Tensid oder eine Tensidmischung,
und
(b) Auftragen bzw. Einbringen der Riechstoffkomposition (a.1) bzw. der Verbindungen(en) / Riechstoffe (a.2) bzw. des Artikels (a.3) auf die zu parfümierende(n) Haare bzw. Haut bzw. Fasern bzw. Oberfläche bzw. in die zu parfümierende Raumluft in einer sensorisch wirksamen Menge, vorzugsweise in einer Menge, die ausreicht, dass der Verbraucher eine oder mehrere Geruchsnoten ausgewählt aus der Gruppe bestehend aus den Noten grün, nitrilig, krautig, frisch, fruchtig, blumig, rosig, holzig, süß, erdig, fettig, metallisch, balsamisch und Iris- und Karottennoten, vorzugsweise wenigstens eine der Geruchsnoten ausgewählt aus der Gruppe bestehend aus den Noten grün, nitrilig, rosig und Iris- und Karottennoten, bevorzugt rosig mit Iris- und Karottennoten, wahrnimmt.

## Claims

1. Use of one or more compound(s) of formula (la) and/or of formula (lb), 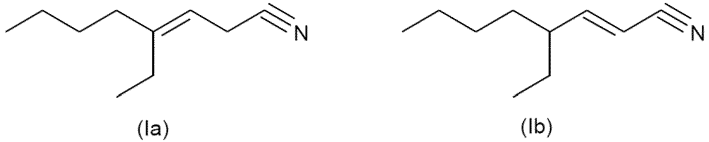 as a fragrance substance.

2. Use according to claim 1 for imparting, modifying and/or enhancing one or more olfactory notes selected from the group consisting of the notes green, nitrile-like, herbaceous, fresh, fruity, floral, rosy, woody, sweet, earthy, greasy, metallic, balsamic and iris and carrot notes, preferably at least one of the olfactory notes selected from the group consisting of the notes green, nitrile-like, rosy and iris and carrot notes, preferably rosy with iris and carrot notes.

3. Fragrance substance composition comprising or consisting of one or more compound(s) of formula (la) and/or formula (lb) 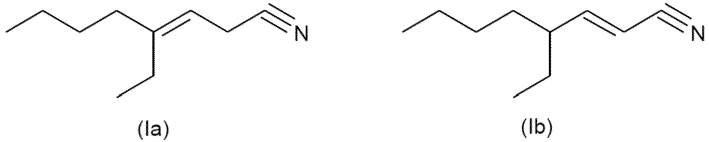 and one, two, three, four, five, six, seven, eight, nine, ten or more further fragrance substance(s), wherein the weight ratio of the total amount of compound(s) of formula (I) to the total amount of further fragrance substance(s) is in the range from 1:1000 to 1:0.1, preferably from 1:1000 to 1:0.5.

4. Fragrance substance composition according to claim 3, wherein the total amount of compound(s) of formula (I), based on the total weight of the fragrance substance composition, is in the range of 0.0001 to 99.9% by weight, preferably 0.001 to 99.5% by weight, most preferably 0.01 to 99% by weight.

5. Fragrance substance composition according to claim 3 or 4, wherein the total amount of the compound(s) of formula (I) in the fragrance substance composition is contained in a sensorially effective amount, preferably in an amount sufficient to impart and/or enhance one or more olfactory notes selected from the group consisting of the notes green, nitrile-like, herbaceous, fresh, fruity, floral, rosy, woody, sweet, earthy, greasy, metallic, balsamic and iris and carrot notes, preferably at least one of the olfactory notes selected from the group consisting of the notes green, nitrile-like, rosy and iris and carrot notes, preferably rosy with iris and carrot notes, and/or to modify one or more olfactory notes of one or of the further fragrance substance(s) of the fragrance substance composition in the direction of one or more of these olfactory notes.

6. Fragrance substance composition according to any of the claims 3 to 5, wherein the or one, several or all of the further fragrance substances (also) impart, modify and/or enhance one or more olfactory notes selected from the group consisting of the notes green, nitrile-like, herbaceous, fresh, fruity, floral, rosy, woody, sweet, earthy, greasy, metallic, balsamic and iris and carrot notes, preferably at least one of the olfactory notes selected from the group consisting of the notes woody, fruity and floral, particularly preferably selected from the group consisting of the notes fruity and floral.

7. Use of a fragrance substance composition according to any of the claims 3 to 6 for imparting, modifying and/or enhancing one or more olfactory notes selected from the group consisting of the notes green, nitrile-like, herbaceous, fresh, fruity, floral, rosy, woody, sweet, earthy, greasy, metallic, balsamic and iris and carrot notes, preferably at least one of the olfactory notes selected from the group consisting of the notes green, nitrile-like, rosy and iris and carrot notes, preferably rosy with iris and carrot notes.

8. Perfumed article, comprising or consisting of
(i) a fragrance substance composition according to any of the claims 3 to 6, and
(ii) one or more further component(s), preferably at least one or more, preferably one, two, three, four, five or more, additive(s), adjuvant(s) and/or active ingredient(s).

9. Perfumed article according to claim 8, wherein the article is selected from the group consisting of cleaning agents and detergents, hygiene or care products, preferably in the field of body and hair care, cosmetics and household, preferably from the group consisting of perfume extracts, eau de parfums, eau de toilettes, aftershaves, eau de colognes, pre-shave products, splash colognes, perfumed refreshing tissues, acidic, alkaline or neutral detergents, textile fresheners, ironing aids, liquid detergents, powdered detergents, laundry pre-treatment agents, fabric softeners, laundry soaps, washing tablets, disinfectants, surface disinfectants, air fresheners, aerosol sprays, waxes and polishes, personal care products, hand creams and lotions, foot creams and lotions, depilatory creams and lotions, aftershave creams and lotions, tanning creams and lotions, hair care products, deodorants, antiperspirants, decorative cosmetic products, candles, lamp oils, incense sticks, insecticides, repellents and fuels.

10. Perfumed article according to claim 8 or 9, wherein component (i) is contained in a sensorially effective amount, preferably in an amount sufficient for a consumer to perceive one or more olfactory notes selected from the group consisting of the notes green, nitrile-like, herbaceous, fresh, fruity, floral, rosy, woody, sweet, earthy, greasy, metallic, balsamic and iris and carrot notes, preferably perceives at least one of the olfactory notes selected from the group consisting of the notes green, nitrile-like, rosy and iris and carrot notes, preferably rosy with iris and carrot notes.

11. Perfumed article according to any of the claims 8 to 10, wherein the total amount of compound(s) of formula (I), based on the total weight of the article, is in the range of 0.00001 to 10% by weight, preferably 0.0001 to 5% by weight, more preferably 0.001 to 2% by weight, more preferably 0.005 to 1% by weight.

12. Method for perfuming an article, comprising or consisting of the following steps:
(a) providing
(a.1) a fragrance substance composition according to any of the claims 3 to 6,
or
(a.2) one or more compound(s) of formula (Ia) and/or of formula (lb) 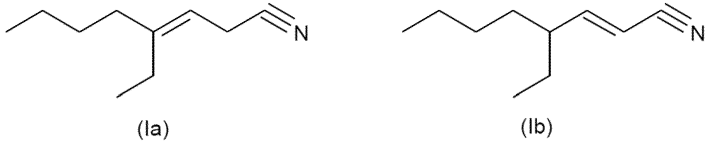 and optionally one, two, three, four, five, six, seven, eight, nine, ten or more further fragrance substance(s), wherein the weight ratio of the total amount of compound(s) of formula (I) to the total amount of further fragrance substance(s) is in the range of 1:1000 to 1:0.1, preferably of 1:1000 to 1:0.5,
and
(b) adding the fragrance substance composition (a.1) or compounds(s)/ fragrance substances (a.2) to the article to be perfumed, in a sensorially effective amount, preferably in an amount sufficient to impart, enhance and/or modify one or more olfactory notes selected from the group consisting of the notes green, nitrile-like, herbaceous, fresh, fruity, floral, rosy, woody, sweet, earthy, greasy, metallic, balsamic and iris and carrot notes, preferably at least one of the olfactory notes selected from the group consisting of the notes green, nitrile-like, rosy and iris and carrot notes, preferably rosy with iris and carrot notes.

13. Method for perfuming hair, skin, textile fibres, surfaces and/or ambient air comprising or consisting of the following steps:
(a) providing
(a.1) a fragrance substance composition according to any of the claims 3 to 6, preferably containing a surfactant or a surfactant mixture,
or
(a.2) one or more compound(s) of formula (Ia) and/or of formula (lb) 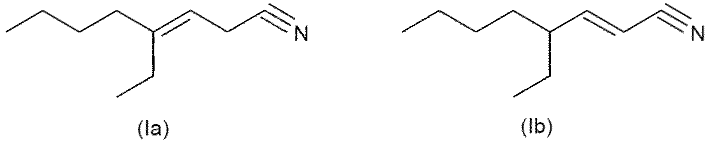 and optionally one, two, three, four, five, six, seven, eight, nine, ten or more further fragrance substance(s), wherein the weight ratio of the total amount of compound(s) of formula (I) to the total amount of further fragrance substance(s) is in the range of 1:1000 to 1:0.1, preferably of 1:1000 to 1:0.5,
and also preferably a surfactant or a surfactant mixture,
or
(a.3) an article according to any of the claims 8 to 11, preferably containing a surfactant or a surfactant mixture,
and
(b) applying or introducing the fragrance substance composition (a.1) or the compound(s)/fragrance substances (a.2) or the article (a.3) to the hair or skin or fibres or surface to be perfumed or into the ambient air to be perfumed, in a sensorially effective amount, preferably in an amount sufficient for the consumer to perceive one or more olfactory notes selected from the group consisting of the notes green, nitrile-like, herbaceous, fresh, fruity, floral, rosy, woody, sweet, earthy, greasy, metallic, balsamic and iris and carrot notes, preferably at least one of the olfactory notes selected from the group consisting of the notes green, nitrile-like, rosy and iris and carrot notes, preferably rosy with iris and carrot notes.

## Revendications

1. Utilisation d'un ou de plusieurs composé(s) de formule (la) et/ou de formule (Ib), 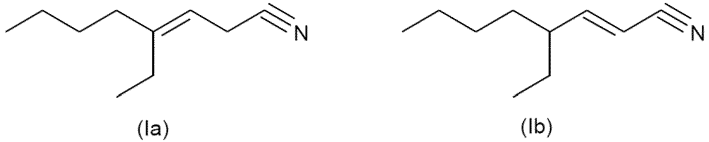 en tant que substance odoriférante.

2. Utilisation selon la revendication 1, pour conférer, modifier et/ou intensifier une ou plusieurs note(s) olfactive(s) choisie(s) dans le groupe constitué par les notes verte, nitrile, herbacée, fraîche, fruitée, fleurie, rosée, boisée, douce, terreuse, graisseuse, métallique, balsamique et les notes d'iris et de carotte, de préférence l'une au moins des notes olfactives choisies dans le groupe constitué par les notes verte, nitrile, rosée et les notes d'iris et de carotte, de préférence rosée avec des notes d'iris et de carotte.

3. Composition de substance odoriférante comprenant ou consistant en un ou plusieurs composé(s) de formule (la) et/ou de formule (Ib) 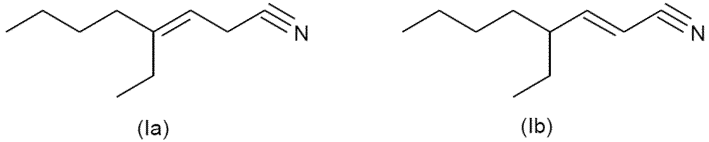 et une, deux, trois, quatre, cinq, six, sept, huit, neuf, dix ou plusieurs autre(s) substance(s) odoriférante(s), dans laquelle le rapport pondéral de la quantité totale de composé(s) de formule (I) à la quantité totale d'autre(s) substance(s) odoriférante(s) se situe dans la plage allant de 1 : 1000 à 1 : 0,1, de préférence de 1 : 1000 à 1: 0,5.

4. Composition de substance odoriférante selon la revendication 3, dans laquelle la quantité totale de composé(s) de formule (I), par rapport au poids total de la composition de substance odoriférante se situe dans la plage allant de 0,0001 à 99,9 % en poids, de préférence de 0,001 à 99,5 % en poids, de manière particulièrement préférée de 0,01 à 99 % en poids.

5. Composition de substance odoriférante selon la revendication 3 ou 4, dans laquelle la quantité totale du ou des composés de formule (I) dans la composition de substance odoriférante est contenue en une quantité sensoriellement efficace, de préférence en une quantité qui est suffisante pour conférer et/ou intensifier une ou plusieurs note(s) olfative(s) choisie(s) dans le groupe constitué par les notes verte, nitrile, herbacée, fraîche, fruitée, fleurie, rosée, boisée, douce, terreuse, graisseuse, métallique, balsamique et les notes d'iris et de carotte, de préférence l'une au moins des notes olfactives choisies dans le groupe constitué par les notes verte, nitrile, rosée et les notes d'iris et de carotte, de préférence rosée avec des notes d'iris et de carotte, et/ou pour modifier une ou plusieurs note(s) olfactive(s) d'une ou bien de la / des autre(s) substance(s) odoriférante(s) de la composition de substance odoriférante en direction d'une ou de plusieurs de ces notes olfactives.

6. Composition de substance odoriférante selon l'une quelconque des revendications 3 à 5, dans laquelle ladite ou bien une, plusieurs ou l'ensemble des autres substances odoriférantes confèrent, modifient et/ou intensifient (elles aussi) une ou plusieurs note(s) olfactive(s) choisie(s) dans le groupe constitué par les notes verte, nitrile, herbacée, fraîche, fruitée, fleurie, rosée, boisée, douce, terreuse, graisseuse, métallique, balsamique et les notes d'iris et de carotte, de préférence l'une au moins des notes olfactives choisies dans le groupe constitué par les notes boisée, fruitée et fleurie, de manière particulièrement préférée choisies dans le groupe constitué par les notes fruitée et fleurie.

7. Utilisation d'une composition de substance odoriférante selon l'une quelconque des revendications 3 à 6, pour conférer, modifier et/ou intensifier une ou plusieurs notes olfactives choisies dans le groupe constitué par les notes verte, nitrile, herbacée, fraîche, fruitée, fleurie, rosées, boisée, douce, terreuse, graisseuse, métallique, balsamique et les notes d'iris et de carotte, de préférence l'une au moins des notes olfactives choisies dans le groupe constitué par les notes verte, nitrile, rosée et les notes d'iris et de carotte, de préférence rosée avec des notes d'iris et de carotte.

8. Article parfumé comprenant ou consistant en
(i) une composition de substance odoriférante selon l'une quelconque des revendications 3 à 6,
et
(ii) un ou plusieurs autres composant(s), de préférence au moins un ou plusieurs, de préférence un, deux, trois, quatre, cinq ou plus, additif(s), excipient(s) et/ou agent(s) actif(s).

9. Article parfumé selon la revendication 8, dans lequel l'article est choisi dans le groupe constitué par les agents de lavage et les agents de nettoyage, les produits d'hygiène ou de soin, de préférence dans le domaine des soins du corps et des cheveux, de la cosmétique et du ménage, de préférence dans le groupe constitué par les extraits de parfum, les eaux de parfum, les eaux de toilettes, les eaux après-rasage, les Eaux de Cologne, les produits de pré-rasage, les Splash Cologne, les lingettes rafraîchissantes parfumées, les agents de nettoyage acides, alcalins et neutres, les produits à rafraîchir les textiles, les aides au repassage, les détergents liquides, les détergents en poudre, les produits de prétraitement de linge, les adousissants de linge, les savons à laver, les pastilles lave-linge, les désinfectants, les désinfectants de surface, les assainisseurs d'air, les aérosols, les cires et polis, les produits de toilette, les crèmes et lotions pour les mains, les crèmes et lotions pour les pieds, les crèmes et lotions dépilatoires, les crèmes et lotions après-rasage, les crèmes et lotions de bronzage, les produits de soins des cheveux, les désodorisants, les antiperspirants, les produits de la cosmétique décorative, les bougies, les huiles de lampe, les bâtonnets d'encens, les insecticides, les répulsifs et les carburants.

10. Article parfumé selon la revendication 8 ou 9, dans lequel le composant (i) est contenu en une quantité sensoriellement efficace, de préférence en une quantité qui est suffisante pour qu'un consommateur aperçoive une ou plusieurs note(s) olfactive(s) choisie(s) dans le groupe constitué par les notes verte, nitrile, herbacée, fraîche, fruitée, fleurie, rosée, boisée, douce, terreuse, graisseuse, métallique, balsamique et les notes d'iris et de carotte, de préférence l'une au moins des notes olfactives choisies dans le groupe constitué par les notes verte, nitrile, rosée et les notes d'iris et de carotte, de préférence rosée avec des notes d'iris et de carotte.

11. Article parfumé selon l'une quelconque des revendications 8 à 10, dans lequel la quantité totale de composé(s) de formule (I), par rapport au poids total de l'article, se situe dans la plage allant de 0,00001 à 10 % en poids, de préférence de 0,0001 à 5 % en poids, de manière particulièrement préférée de 0,001 à 2 % en poids, de manière encore plus préférée de 0,005 à 1 % en poids.

12. Procédé destiné à parfumer un article, comprenant ou se composant des étapes suivantes consistant à:
(a) fournir
(a.1) une composition de substance odoriférante selon l'une quelconque des revendications 3 à 6,
ou
(a.2) un ou plusieurs composé(s) de formule (la) et/ou de formule (Ib) 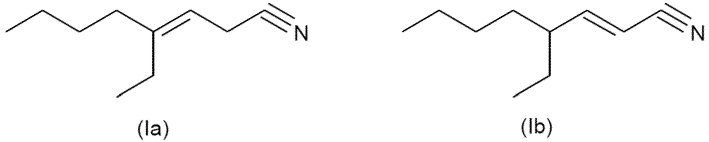 et, en option, une, deux, trois, quatre, cinq, six, sept, huit, neuf, dix ou plusieurs autre(s) substance(s) odoriférante(s), dans lequel le rapport pondéral de la quantité totale de composé(s) de formule (I) à la quantité totale d'autre(s) substance(s) odoriférante(s) se situe dans la plage allant de 1 : 1000 à 1 : 0,1, de préférence de 1 : 1000 à 1: 0,5.
et
(b) ajouter la composition de substance odoriférante (a.1) ou le/les composé(s) / substances odoriférantes (a.2) à l'article à parfumer, en une quantité sensoriellement efficace, de préférence en une quantité qui est suffisante pour conférer, intensifier et/ou modifier une ou plusieurs note(s) olfative(s) choisie(s) dans le groupe constitué par les notes verte, nitrile, herbacée, fraîche, fruitée, fleurie, rosée, boisée, douce, terreuse, graisseuse, métallique, balsamique et les notes d'iris et de carotte, de préférence l'une au moins des notes olfactives choisies dans le groupe constitué par les notes verte, nitrile, rosée et les notes d'iris et de carotte, de préférence rosée avec des notes d'iris et de carotte.

13. Procédé destiné à parfumer des cheveux, la peau, des fibres textiles, des surfaces et/ou de l'air ambiant, comprenant ou se composant des étapes suivantes consistant à:
(a) fournir
(a.1) une composition de substance odoriférante selon l'une quelconque des revendications 3 à 6, de préférence contenant un tensioactif ou un mélange de tensioactifs,
ou
(a.2) un ou plusieurs composé(s) de formule (la) et/ou de formule (Ib) 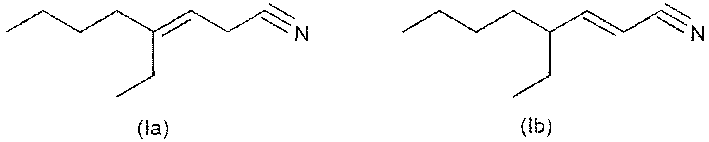 et, en option, une, deux, trois, quatre, cinq, six, sept, huit, neuf, dix ou plusieurs autre(s) substance(s) odoriférante(s), dans lequel le rapport pondéral de la quantité totale de composé(s) de formule (I) à la quantité totale d'autre(s) substance(s) odoriférante(s) se situe dans la plage allant de 1 : 1000 à 1 : 0,1, de préférence de 1 : 1000 à 1: 0,5, et, en outre, de préférence un tensioactif ou un mélange de tensioactifs,
ou
(a.3) un article selon l'une quelconque des revendications 8 à 11, de préférence contenant un tensioactif ou un mélange de tensioactifs,
et
(b) appliquer ou bien introduire la composition de substance odoriférante (a.1) ou le/les composé(s) / substances odoriférantes (a.2) ou bien l'article (a.3) aux cheveux ou bien à la peau ou bien aux fibres ou bien à la surface à parfumer ou bien dans l'air ambiant à parfumer, en une quantité sensoriellement efficace, de préférence en une quantité qui est suffisante pour que le consommateur aperçoive une ou plusieurs note(s) olfactive(s) choisie(s) dans le groupe constitué par les notes verte, nitrile, herbacée, fraîche, fruitée, fleurie, rosée, boisée, douce, terreuse, graisseuse, métallique, balsamique et les notes d'iris et de carotte, de préférence l'une au moins des notes olfactives choisies dans le groupe constitué par les notes verte, nitrile, rosée et les notes d'iris et de carotte, de préférence rosée avec des notes d'iris et de carotte.
